# EUROPEAN PATENT APPLICATION

(11) **EP 3 156 065 A1**
(43) Date of publication of application: **19.04.2017**
(21) Application number: 15806618.3
(22) Date of filing: 27.05.2015
(51) Int. Cl.: A61K 38/17, A61K 9/06, A61K 38/00, A61K 47/06, A61K 47/10, A61K 47/44, A61P 17/02

(54) **HYDROLYZED FIBROIN-CONTAINING OINTMENT AND PRODUCTION METHOD THEREFOR**

(30) Priority: 12.06.2014 JP 2014121441
(71) Applicant: Adan Co., Ltd., Amami-shi, Kagoshima 894-0007 (JP); Kagoshima University, Kagoshima-shi, Kagoshima 890-8580 (JP)
(72) Inventor: NISHI Hirohito, Amami-shi Kagoshima 894-0007 (JP); KANEKURA Takuro, Kagoshima-shi Kagoshima 890-8580 (JP)
(74) Representative: Zoli, Filippo
(86) International application number: PCT/JP2015/065196
(87) International publication number: WO 2015/190292

(57) **Abstract**

A topical agent that is a preparation containing hydrolyzed silk and is applied to a wound site for use in wound healing, an object thereof being to have an effect of promoting wound healing, and to satisfy practical application conditions with regard to an irritative property, stability, handling ease, and the like, wherein the topical agent contains hydrolyzed fibroin, an oil-based component, and a water-based component. The topical agent of the present invention is preferably an ointment or a cream. The topical agent contains hydrolyzed fibroin as an active ingredient in an amount of 1 to 40 wt%, and preferably does not contain a surfactant.

## Description

### TECHNICAL FIELD

The present invention pertains to a topical agent containing a hydrolyzed fibroin, and particularly pertains to an ointment containing a hydrolyzed fibroin and having a wound healing promotion effect and to a method for manufacturing the same.

Hydrolyzed silk is a mixture of protein hydrolysates obtained by treating natural silk fibers produced by silkworms (Bombyx mori) with acid, alkali, a concentrated salt solution, or the like. So-called hydrolyzed silk is a generic term for hydrolysates of fibroin and sericin, which are proteins that constitute silk fibers.

Hydrolyzed silk is known to have various physiological effects. For example, patent document 1 discloses that a hydrogel having rubberlike elasticity and capable of maintaining long-lasting water retention characteristics can be obtained by exposing a mixture of polyvinyl alcohol and hydrolyzed silk protein to radiation, and that the material can be used as a wound dressing.

An object of the invention of patent document 1 is to improve the heat resistance and strength of a hydrogel fabricated by irradiating polyvinyl alcohol with radiation, and patent document 1 discloses that a hydrogel excelling in strength and water retention characteristics is obtained by adding hydrolyzed silk protein to polyvinyl alcohol. Patent document 1 also describes that the addition of hydrolyzed silk protein is effective for the biocompatibility of the hydrogel.

Furthermore, patent document 2 discloses a topical agent for skin containing L-ascorbic acid, derivatives thereof, and hydrolyzed silk protein. This topical agent for skin has an effect of preventing and improving pigmentation, drying, rough skin, and the like, and also imparts the skin with a comprehensive cosmetic effect. The invention of patent document 2 blends hydrolyzed silk proteins with L-ascorbic acid, which has a whitening effect, and sets the pH within a range of from 5 to 8, and as a result, the whitening effect is synergistically strengthened, and a topical agent for skin that is stable and presents no problems in terms of safety can be obtained.

On the other hand, patent document 3 discloses that an undecomposed silk fibroin can be stored for a long period of time in an undecomposed state by using a prescribed concentration of calcium ions or magnesium ions. Patent document 3 also describes that because the undecomposed silk fibroin has an action of promoting the growth of fibroblasts derived from human skin, an agent containing the undecomposed silk fibroin can be used in an ointment or cream type wound dressing, wound healing material, cosmetics, or the like.

Furthermore, patent document 4 discloses that a composite obtained by coating the surface of silicone rubber with silk protein (silk fibroin) exhibits a high level of cellular adhesion and proliferation. Patent document 4 discloses that by imparting silk fibroin on the surface of silicone rubber, which is a material that is inactive and non-hydrophilic, the surface becomes hydrophilic, and the cellular adhesion and cellular proliferation on the surface are improved.

### PRIOR ART DOCUMENTS

### Patent Documents

Patent Document 1: Japanese Unexamined Patent Application Publication No. 2000-169736
Patent Document 2: Japanese Unexamined Patent Application Publication No. 2007-277119
Patent Document 3: Japanese Unexamined Patent Application Publication No. 2003-2898
Patent Document 4: Japanese Unexamined Patent Application Publication No. H03-123561

### SUMMARY OF THE INVENTION

### Problem to be Solved by the Invention

As described above, hydrolyzed silk is known to excel in biocompatibility and to have an esthetic effect on the skin. However, the effect of hydrolyzed silk on skin damage (wounds) was not known. Moreover, a covering made from silk fibroin and silk fibroin film were known to promote the adhesion and proliferation of fibroblasts, but the effectiveness of such in the wound healing process was not known. In addition, absolutely no examinations had been performed on the appropriate formulation, concentration, stability, and the like when applied to a wound.

In light of the above, an object of the present invention is to provide a topical agent that is a preparation containing hydrolyzed silk, the topical agent thereof having an effect of promoting wound healing, and satisfying practical application conditions with regard to the irritative property, stability, handling ease, and the like.

### MEANS FOR SOLVING THE PROBLEM

While examining measures to solve the abovementioned problems, the inventors discovered that when an ointment having sericin-free hydrolyzed silk (hydrolyzed fibroin) as a component is applied to a wound site on a mouse, wound healing is better facilitated compared to the use of an ointment that does not contain hydrolyzed fibroin as a component. The inventors also discovered that hydrolyzed fibroin itself has an interfacial activating effect, and therefore a preparation can be stably formed using hydrolyzed fibroin, an oil-based component, and a water-based component without using a surfactant, and that a preparation having a high practical value and a low irritative property and excelling in stability and handling ease can be obtained, and the present invention was thereby accomplished.

Namely, the present invention pertains to a topical agent containing hydrolyzed fibroin, an oil-based component, and a water-based component, and which can be applied to a wound site and used for wound healing.

The topical agent of the present invention is preferably an ointment or a cream.

Moreover, the content of hydrolyzed fibroin that is the active ingredient in the topical agent of the present invention is preferably 1 to 40 wt%.

Furthermore, the topical agent preferably does not contain a surfactant. The oil-based component is preferably one or more of a vegetable oil, paraffin or sesame oil, olive oil, shea butter, rice germ oil, and squalane oil, and the water-based component preferably contains polyhydric alcohol.

In addition, the present invention pertains to a method for manufacturing an ointment preparation, the method including the steps of:
(1) removing sericin from a silk material, and then heating the silk material for 40 to 70 minutes in a 40 to 60 wt% aqueous solution of calcium chloride or lithium bromide;
(2) desalinating the solution obtained in (1) by dialysis;
(3) adjusting the concentration of the solution obtained in (2), and then leaving the resulting solution stationary to obtain a hydrolyzed fibroin paste; and
(4) agitating and mixing the hydrolyzed fibroin paste obtained in (3) and an oily base.

The topical agent of the present invention is preferably a wound healing promoter.

### EFFECT OF THE INVENTION

The present invention provides a topical agent having a wound healing promotion effect, and particularly provides an ointment or cream having a wound healing promotion effect. The topical agent of the present invention contains hydrolyzed fibroin, which is a natural ingredient, as an active ingredient, and has a wound healing promotion effect, and at the same time, the present invention can provide a topical agent that does not contain a surfactant, has a low irritative property, and excels in stability and handling ease. The advanced wound healing promotion effect of the present invention was confirmed by comparing a full-layer deficit wound to which an ointment of the present invention was applied with a control group, whereby significantly good results in terms of the formation of granulation tissue, wound thickness, and re-epithelialization were exhibited.

### BRIEF DESCRIPTION OF THE DRAWINGS

FIG. 1 shows HE stained images of cross-sections of mice wounds to which an ointment of an embodiment of the present invention and an ointment of a comparative example were applied.
FIG. 2 is a graph showing the surface area of granulation tissue at mice wounds to which the ointment of the embodiment of the present invention and the ointment of the comparative example were applied.
FIG. 3 is a graph showing the thickness at the center of mice wounds to which the ointment of the embodiment of the present invention and the ointment of the comparative example were applied.
FIG. 4 is a graph showing the re-epithelialization rate of mice wounds to which the ointment of the embodiment of the present invention and the ointment of the comparative example were applied.

### MODE FOR CARRYING OUT THE INVENTION

The topical agent of the present invention contains hydrolyzed fibroin as an active ingredient, and the hydrolyzed fibroin, an oil-based component, and a water-based component are mixed together. As the hydrolyzed fibroin, one having a number-average molecular weight of 10000 to 100000 and a weight-average molecular weight of 50000 to 150000 can be used. Here, the average molecular weights were measured by gel filtration. A hydrolyzed fibroin having a number average molecular weight of 20000 to 30000 and a weight-average molecular weight of 80000 to 95000 is more preferred. Furthermore, the use of hydrolyzed fibroin having a molecular weight distribution that is a nearly normal distribution is preferred. While not bound to a specific theory, when a hydrolyzed fibroin having a wide ranging molecular weight distribution is used, the hydrolyzed fibroin materials of each molecular weight act in a multi-tiered manner, and as a result, good wound healing can be obtained. As a guideline for molecular weight distribution, for example, hydrolyzed fibroin having a Mw/Mn of 2.0 to 5.0 can be used. The content amount of the hydrolyzed fibroin in the topical agent is 1 to 40 wt%, preferably 1 to 20 wt%, and more preferably 2 to 10 wt%.

As the hydrolyzed fibroin, a commercially available product may be used, or the hydrolyzed fibroin may be prepared and used.

Fibroin is a protein which constitutes silk together with sericin, and hydrolyzed fibroin is a protein decomposition product (polypeptide) obtained by removing sericin from silk to obtain only fibroin, and then subjecting the fibroin to hydrolysis.

The oil-based component is the base ingredient of the topical agent, and examples include vegetable oil, a paraffin component, hydrocarbons, esters, and the like. Vegetable oil is an oil which has a plant as the raw material, and specific examples include soybean oil, rice oil, canola oil, cottonseed oil, sesame oil, safflower oil, almond oil, castor oil, olive oil, cacao oil, camellia oil, sunflower seed oil, palm oil, linseed oil, perilla oil, shea oil, palm oil, jojoba oil, grapeseed oil, avocado oil, and the like, but the vegetable oil is not limited to these examples. Of these, olive oil and sesame oil are preferred because they contain a relatively large amount of oleic acid, which does not easily oxidize.

The paraffin component means an ester of a higher fatty acid and higher alcohol. Specific examples include beeswax, spermaceti, lanoline, carnauba wax, candelilla wax, Japan wax, and the like, but the paraffin is not limited thereto. Of these, beeswax is preferred because the melting point is high when it is used.

As the oil-based component, in addition to the abovementioned vegetable oil and paraffin component, hydrocarbons or ester oil may be contained. Hydrocarbons are compounds consisting of carbon and hydrogen, and specific examples include liquid paraffin, squalene, squalane, α-olefin, microcrystalline wax, ceresin, paraffin, white petrolatum, yellow petrolatum, and the like, but hydrocarbon is not limited thereto.

Ester oils are esters of alcohol and fatty acid, and specific examples include isopropyl palmitate, octyl palmitate, 2-ethylhexyl palmitate, isopropyl myristate, isononyl isononanoate, medium-chain triglyceride, caprylic/capric triglyceride, and the like.

The abovementioned oil-based component is selected primarily from the perspective of product stability and handling ease (viscosity, extensibility, and the like). For example, the combined use of a vegetable oil and paraffin component is preferred, and the combined use of a vegetable oil and beeswax is particularly preferred. The content amount of the vegetable oil is 15 to 35%, and preferably 20 to 30%. The content amount of the paraffin component is 1 to 15%, and is preferably 5 to 10%. When the content amounts thereof are within these ranges and the vegetable oil and paraffin component are mixed with the other components, an ointment having an appropriate amount of viscosity and extensibility is obtained.

As the water-based component, in addition to water, other examples include polyhydric alcohols, lower alcohols, and the like, and polyhydric alcohol is preferably contained along with water. Examples of polyhydric alcohols include glycerin, propylene glycol, 1,3-butylene glycol, polyethylene glycol, 1,2-pentanediol, 1,5-pentanediol, 1,6-hexanediol, mannitol, sorbitol, and the like, and 1,3-butylene glycol is preferred. Examples of lower alcohols include ethanol, methanol, and the like. The water-based component is contained at an amount of 40 to 70%, and preferably 55 to 65%.

In addition to the abovementioned hydrolyzed fibroin, oil-based component, and water-based component, the topical agent may contain various well-known additives. Examples of such additives include a surfactant, antiseptic (antibacterial agent, antifungal agent), pH adjuster, antioxidant agent, stabilizer, thickening agent, and the like. When these additives are included, the total amount of the additives can be 0.1 to 1%, and is more preferably 0.2 to 0.5%. However, with the topical agent of the present invention, because the hydrolyzed fibroin itself, which is an active ingredient, has a surface activating effect, a stable ointment or cream can be obtained without the use of a surfactant.

The topical agent of the present invention is preferably a formulation that can be applied through coating, and more specifically is an ointment or cream (hereinafter, unless otherwise described, a cream shall also be included as an "ointment"). If an ointment is emulsified, the emulsification form may be water-in-oil or oil-in-water. Moreover, other than emulsification, the hydrolyzed fibroin in the preparation may be present in a colloid form or a dispersed state in the base. In either case, the base can be a base obtained through a well-known base composition.

Examples of wounds to which the topical agent of the present invention can be applied include lacerations, abrasions, surgical incisions, skin ulcerations, burn injuries, and the like. Furthermore, the topical agent of the present invention is thought to have an effect of promoting both granulation formation and re-epithelialization in the wound healing process, and can be applied to both an epidermis deficit wound where the epidermis is injured, and a full-layer deficit wound for which the epidermis and the dermis are injured.

The topical agent of the present invention is applied to a wound site, and the amount that is applied can be appropriately selected from the well-known range, and is not particularly limited. However, for example, an amount of 0.01 mL to 1 mL and preferably 0.01 mL to 0.20 mL can be applied to a circular wound having a diameter of 5 mm. The method of application may be application directly to the wound surface, or coating a gauze or film with the ointment and then adhering the gauze or film such that the coated surface contacts the wound surface. If the ointment is directly applied to the wound surface, the entire ointment that has been applied is then preferably covered with a stretchable polyurethane film, gauze, or the like to maintain the environment of the wound surface. Furthermore, the ointment of the present invention may also be used in combination with various well-known wound dressings.

### <Manufacturing Method>

### (1) Manufacturing the Hydrolyzed Fibroin

Manufacturing of the hydrolyzed fibroin can be done, for example, by removing sericin from natural silk (threads, cocoons) through an alkaline treatment, solubilizing and decomposing the remaining fibroin by heating in a 30 to 70 wt% concentrated salt solution, and then removing the salt and low molecular weight substance by dialysis and ultrafiltration to obtain hydrolyzed fibroin.

As the raw material, silkworm cocoons, commercially available scoured thread, unscoured thread, and the like can be used. If cocoons or unscoured thread are used, first the sericin is removed by a well-known method such as alkaline scouring (soap, sodium carbonate), enzymatic scouring, and the like. Next, silk from which the sericin has been removed is inserted into a concentrated solution of calcium chloride, lithium bromide, or the like, and dissolved by heating for 40 to 70 minutes. Next, the obtained solution is inserted into a dialysis membrane, and dialysis with respect to water is performed to desalinate the solution, and at the same time, the low molecular components are removed.

Next, the solution after desalination is heated and concentrated to a prescribed concentration, a prescribed amount of antiseptic and polyhydric alcohol are added, and the mixture is left stationary for about 5 to 20 days at a temperature of 18°C to 30°C to obtain a hydrolyzed fibroin paste.

### (2) Manufacturing an Ointment

The hydrolyzed fibroin paste obtained as described above is agitated with an antiseptic. Next, a separately dissolved and mixed vegetable oil and paraffin component are added, and the mixture is further agitated and mixed to obtain an ointment.

### EXAMPLES

### [Example 1]

### (1) Preparation of a Hydrolyzed Fibroin

An amount of 5000 cc of water and 5000 g of calcium chloride were mixed, and while the mixture was boiled, 1 kg of scoured thread was inserted and heated for 50 minutes. Next, 3000 cc of water was added, the mixture was once again boiled, heating was completed, and a fibroin dissolved substance was obtained.

The fibroin dissolved substance was passed through a stainless steel strainer and then inserted into a dialysis membrane (cutoff molecular weight of 14000, pore size of 50Å), and then dialysis was performed with flowing water for 4 days, and the substance was desalinated.

After desalination, the substance was heated, and moisture was caused to evaporate until the weight became about 58%.

After heating, an antiseptic (a dissolved solution of 1 kg of methyl paraben in 36 L of 1,3-butylene glyocol) was added at a temperature of about 60°C such that the antiseptic became 0.4% and agitated.

Coarse substances were then removed by passing the mixture through a stainless steel strainer, after which the mixture was transferred to a stainless steel container with a lid and then left stationary for approximately 10 days at a room temperature of 18°C to 30°C, and a hydrolyzed fibroin paste was obtained. When the molecular weight of the hydrolyzed fibroin paste was measured through gel filtration, the number-average molecular weight (Mn) was 23750, the weight-average molecular weight (Mw) was 86750, and the Mw/Mn was 3.65.

### (2) Preparation of a Hydrolyzed Fibroin Ointment

Twenty-seven grams of an antiseptic were added to 400 g of the hydrolyzed fibroin paste obtained in (1) above, and the material was agitated for 2 minutes using a food processor.

An amount of 200 cc of olive oil and 50 g of beeswax were mixed in hot water, and then inserted four separate times into the abovementioned food processor and agitated for 5 to 10 minutes, and a hydrolyzed fibroin ointment was obtained.

The composition of the hydrolyzed fibroin ointment obtained as described above was as follows.

**[Table 1]**

| Hydrolyzed Fibroin Ointment | |
|---|---|
| Component | % |
| Olive oil | 27.6 |
| Beeswax | 7.58 |
| 1,3-butylene glycol | 12.57 |
| Propyl paraoxybenzoate | 0.11 |
| Methyl paraoxybenzoate | 0.23 |
| Hydrolyzed fibroin | 4 |
| Water | 47.91 |
| Total | 100.0 |

### <Antiseptic and Antifungal Tests>

Antiseptic and antifungal tests with respect to (1) standard bacterial mixed bacteria, (2) wastewater mixed bacteria, and (3) fungus mixed bacteria were performed on the hydrolyzed fibroin ointment that was obtained as described above. Each of the mixed bacteria was as follows.

**[Table 2]**

| | | |
|---|---|---|
| (1) Standard Bacterial Mixed Bacteria: | *Escherichia coli* | NBRC 3972 |
| | *Pseudomonas aeruginosa* | NBRC 13275 |
| | *Staphylococcus aureus* | NBRC 13276 |
| (2) Wastewater Mixed Bacteria: | Kitchen wastewater filtrate | |
| (3) Fungus Mixed Bacteria: | *Aspergillus brasiliensis* | NBRC 9455 |
| | *Candida albicans* | NBRC 1594 |
| | *Penicillium citrinum* | NBRC 6352 |
| | *Aureobasidium pullulans* | NBRC 6353 |

Twenty grams of hydrolyzed fibroin ointment was placed in respective sterilized vial containers, and each test bacterial solution was inoculated to each sample at an amount of 1% (0.2 mL). The standard bacterial mixed bacteria and the wastewater mixed bacteria were maintained at 30°C, the fungus mixed bacteria was maintained at 25°C, and the viable bacteria count was measured after 7, 14, and 21 days.

The viable count was measured using the SCDLP agar media mix dilution method for the bacteria and the GPLP agar media mix dilution method for the fungus.

The viable count when the test was started (Table 3), the number of inoculum organisms (Table 4), and the change over time of the viable count after inoculation (Table 5) are as shown below.

**[Table 3]**

| Sample | pH | Viable Count (number/g) | |
|---|---|---|---|
| | | Bacteria | Fungus |
| Hydrolyzed fibroin ointment | 6.0 | <10¹ | <10¹ |

**[Table 4]**

| Inoculum Organism | Viable Count of Inoculum Organism Solution (number/mL) | Viable Count in Sample (number/g) |
|---|---|---|
| Standard Bacterial Mixed Bacteria | 1.4×10⁸ | 1.4×0⁶ |
| Wastewater Mixed Bacteria | 3.1×10⁷ | 3.1×10⁵ |
| Fungus Mixed Bacteria | 1.7×10⁷ | 1.7×10⁵ |

**[Table 5]**

| Sample | Inoculum Organism | Viable Count (number/g) | | |
|---|---|---|---|---|
| | | 7 days | 14 days | 21 days |
| Hydrolyzed Fibroin Ointment | Standard Bacterial Mixed Bacteria | <10¹ | <10¹ | <10¹ |
| | Wastewater Mixed Bacteria | <10¹ | <10¹ | <10¹ |
| | Fungus Mixed Bacteria | 1.3×10⁴ | 1.2×10⁴ | 1.2×10⁴ |

As noted in the above tables, the hydrolyzed fibroin ointment of the present invention exhibited a good antiseptic effect. While the viable bacterial count of the fungus was somewhat high, no propagation occurred, and therefore it was determined that no obstacles with respect to practical application exist.

### <Wound Healing Tests>

### Test animal: six mice (BALB/c, female, 8-12 weeks)

Medicinal agent targeted for testing: hydrolyzed fibroin ointment (Example 2, test group) and ointment not containing hydrolyzed fibroin (Comparative Example, control group). The ointment obtained in Example 1 was used as the hydrolyzed fibroin ointment, and the composition of the ointment of the Comparative Example is as shown in the following table.

**[Table 6]**

| Ointment Not Containing Hydrolyzed Fibroin | |
|---|---|
| Component | % |
| Olive oil | 70.28 |
| Beeswax | 19.30 |
| 1,3-butylene glycol | 10.14 |
| Propyl paraoxybenzoate | 0.28 |
| Total | 100.0 |

Test Method: The hair from the dorsal region of the mouse was shaved away, and then using a trepan (diameter 5 mm), full-layer deficit wounds were created in a total of four places, two locations each on the right and left sides. A 21G syringe needle was then used to fill the wound sites with 0.03 mL of the ointment of Example 2 or the ointment of the Comparative Example, and the wound sites were covered entirely with an adhesive urethane film (Multi Fix from Alcare Co., Ltd.). The torso of the mouse was then wrapped with a self-adhering elastic bandage to prevent the urethane film from falling off. Note that for each mouse, the ointment of Example 2 was applied to two wound sites on either the right or the left sides, and the ointment of the Comparative Example was applied to the remaining two wound sites.

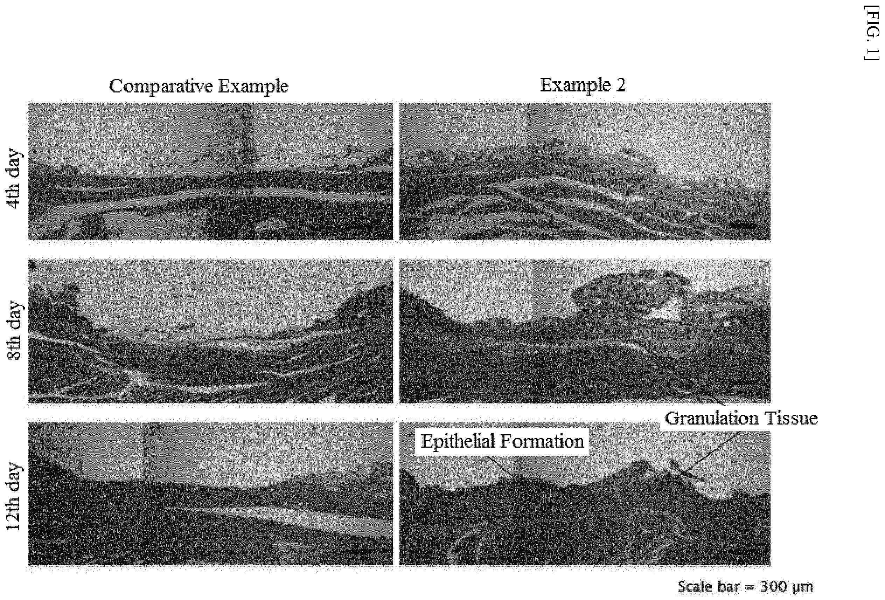

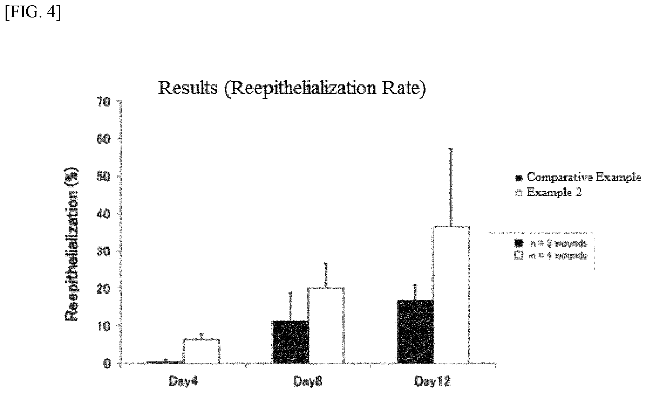

On the 4th day, the 8th day, and the 12th day, the wound sites of two mice each were visually observed, the overall wound sites were sampled, and cross-sectional slices of the wound sites were HE stained. Image J software was then used to measure the surface area of granulation formation, the thickness at the wound center, and the regeneration rate of the epidermis from the images of the HE stained tissue slices. The surface area of granulation formation and the wound thickness were measured values, and the re-epithelialization rate was calculated by (length of the epidermis of the epithelialized portion ÷ the diameter of the overall wound)×100 = (%). The results are shown in FIG. 1 to FIG. 4.

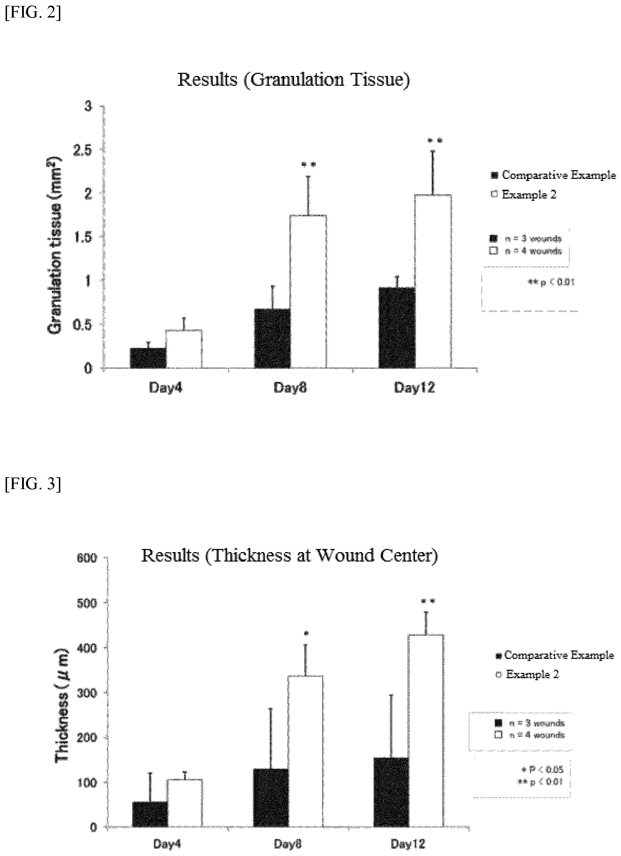

As shown by FIG. 2 to FIG. 4, the group to which the hydrolyzed fibroin ointment was applied excelled in each of the areas of granulation tissue surface area, thickness at the wound center, and re-epithelialization at the 4^{th}, 8^{th}, and 12^{th} days compared to the control group. In other words, it was confirmed that the ointment of the present invention containing hydrolyzed fibroin facilitates both the formation of granulation tissue and the regeneration of epidermis.

## Claims

1. A topical agent which is applied to a wound site for use in wound healing, the topical agent comprising: a hydrolyzed fibroin; an oil-based component; and a water-based component.

2. The topical agent according to claim 1, the topical agent being an ointment or a cream.

3. The topical agent according to claim 1 or claim 2, wherein the hydrolyzed fibroin is an active ingredient, and the topical agent contains the hydrolyzed fibroin in an amount of 1 to 40 wt%.

4. The topical agent according to any one of claims 1 to 3, wherein the topical agent does not contain a surfactant.

5. The topical agent according to any one of claims 1 to 4, wherein the oil-based component contains one or more of a vegetable oil, paraffin or sesame oil, olive oil, shea butter, rice germ oil, and squalane oil.

6. The topical agent according to any one of claims 1 to 5, wherein the water-based component comprises a polyhydric alcohol.

7. A method for manufacturing an ointment preparation, the method comprising the steps of:
(1) removing sericin from a silk material, and then heating the silk material for 40 to 70 minutes in a 40 to 60 wt% aqueous solution of calcium chloride or lithium bromide;
(2) desalinating the solution obtained in (1) by dialysis;
(3) adjusting the concentration of the solution obtained in (2), and then leaving the resulting solution stationary to obtain a hydrolyzed fibroin paste; and
(4) agitating and mixing the hydrolyzed fibroin paste obtained in (3) and an oily base.

8. The method according to claim 7, wherein the ointment preparation is a wound healing promoter.
